# EUROPÄISCHE PATENTSCHRIFT

(11) **EP 3 221 504 B1**
(45) Veröffentlichungstag und Bekanntmachung des Hinweises auf die Patenterteilung: **23.08.2023**
(21) Anmeldenummer: 15797280.3
(22) Anmeldetag: 11.11.2015
(51) Int. Cl.: F25D 29/00, A61B 5/00, A47L 15/42, F24C 7/08, D06F 34/32, A61B 5/024, D06F 33/00

(54) **HAUSGERÄT MIT EINER BERÜHRUNGSEMPFINDLICHEN BEDIENEINRICHTUNG SOWIE VERFAHREN ZU SEINEM BETRIEB**
DOMESTIC APPLIANCE HAVING A TOUCH-SENSITIVE OPERATOR CONTROL DEVICE, AND METHOD FOR OPERATING THE SAME
APPAREIL ÉLECTROMÉNAGER PRÉSENTANT UN DISPOSITIF DE COMMANDE SENSIBLE AU CONTACT ET PROCÉDÉ POUR SON FONCTIONNEMENT

(30) Priorität: 17.11.2014 DE 102014223366
(43) Veröffentlichungstag der Anmeldung: 27.09.2017
(73) Patentinhaber: BSH Hausgeräte GmbH, 81739 München (DE)
(72) Erfinder: NEITZEL, Thomas, 10785 Berlin (DE)
(86) Internationale Anmeldenummer: PCT/EP2015/076304
(87) Internationale Veröffentlichungsnummer: WO 2016/078982

(56) Entgegenhaltungen:
- WO-A1-2014/088333
- WO-A1-2014/104775
- US-A1- 2013 120 114

## Beschreibung

Die Erfindung betrifft ein Hausgerät mit einer berührungsempfindlichen Bedieneinrichtung sowie ein Verfahren zu seinem Betrieb. Die Erfindung betrifft insbesondere ein solches Hausgerät mit einer Steuereinrichtung und einer berührungsempfindlichen Bedieneinrichtung sowie ein bevorzugtes Verfahren zu seinem Betrieb.

Zur Bedienung von Computern und Smartphones ist es in den letzten Jahren zunehmend beliebter geworden, eine berührungsempfindliche Anzeige- und Bedieneinrichtung zu verwenden. Eine solche berührungsempfindliche Anzeige- und Bedieneinrichtung, die eine Anzeigevorrichtung mit einer Bedieneinrichtung kombiniert und im Folgenden auch als "Touchscreen" bezeichnet wird, ermöglicht prinzipiell eine angenehme und rasche Bedienung des betreffenden Gerätes. Dementsprechend gibt es bereits zahlreiche Patentanmeldungen und Patente betreffen Touchscreens, beispielsweise die US-Patente Nr. 5,559,301, 6,130,665 und US 6,542,171.

Es gibt mittlerweile auch Hausgeräte mit einem Touchscreen. Ein solches Hausgerät ist beispielsweise in der WO 2014/104775 A1 offenbart. Allerdings kann die Steuerung eines Hausgerätes über einen Touchscreen dann sehr schwierig sein, wenn die Anzahl von ausführbaren Programmen und Einstellmöglichkeiten von Parametern sehr groß sind. Bei einer komplexen Hierarchie von Programmen und Einstellmöglichkeiten kann ein Benutzer des Hausgerätes sehr leicht verwirrt werden und den Überblick über die Steuerung verlieren. Dies ist insbesondere der Fall, wenn für bestimmte Programmschritte viele einzelne Einstellmöglichkeiten gegeben sind. Aber gerade die Möglichkeit der Eingabe von optimalen Einstellungen kann einen sehr effizienten Betrieb eines Hausgerätes ermöglichen. Neben einem in Hinblick auf den Verbrauch an Energie und Einsatzmaterialen wie Wasser, Reinigungsmittel etc. effizienteren Betrieb des Hausgerätes kann der Betrieb auch besonders benutzerfreundlich gestaltet werden, wenn die Eingabe von optimalen Einstellungen möglich ist.

Das Display, d.h. der Touchscreen, begrenzt jedoch den Bereich von Bewegungen, welcher für die Einstellung von Parametern benutzt werden kann, und begrenzt auch die Anzahl der Parameter, die durch Verwendung eines Displays eingestellt werden können. Dieses erfordert eine tiefe Hierarchie von Menüs und Fenstern für die Zwecke der Einstellung von Parametern, die für ein im Hausgerät durchzuführendes Programm verwendet werden sollen. Damit verzögert sich jedoch die Steuerung eines Programms oder aber es werden von einem vielleicht nicht sehr geduldigen Benutzer nicht optimierte Verfahrensweisen vorgegeben, so dass der Betrieb des Hausgerätes nicht optimal erfolgt. Es ist daher erwünscht, die Bedienung eines Hausgerätes mit einem Touchscreen möglichst effizient und angenehm zu gestalten.

Jeder Benutzer eines Hausgerätes hat zudem unterschiedliche Vorlieben und Wünsche bezüglich der Betriebsweise eines Hausgerätes. Diese Vorlieben und Wünsche können sich auf die Gestaltung und Funktionsweise einer berührungsempfindlichen Bedieneinrichtung beziehen, insbesondere aber auch auf den Betrieb des Hausgerätes hinsichtlich des eigentlichen Einsatzzweckes des Hausgerätes. Wünschenswert wäre es, wenn derartige Vorlieben und Wünsche ohne großes Zutun eines Benutzers berücksichtigt werden könnten.

Überdies gibt es selbst bei einem bestimmten Benutzer zeitlich variierende emotionale und gesundheitliche Zustände, welche einen Einfluss auf eine sinnvolle Benutzung und Betriebsweise des Hausgerätes haben. Ein Eingehen auf die individuellen Bedürfnisse eines Benutzers würde die Bedienerfreundlichkeit und Sicherheit des Betriebes eines Hausgerätes verbessern. Insgesamt würde damit auch die Betriebslebensdauer eines Hausgerätes im Allgemeinen verlängert werden.

Die Veröffentlichung US 8 398 546 B2 beschreibt ein System für die Überwachung und die Steuerung des Körpergewichtes und von anderen physiologischen Bedingungen einschließlich Das System stellt eine Rückmeldung zur Verfügung für das Ziel der Gewichtsreduzierung bei einem Individuum. Hierzu ist ein tragbarer Sensor für die Messung von Daten und eine hiermit in elektronischer Kommunikation stehende Verarbeitungseinheit vorhanden. Auf der Basis von aus den aufgenommenen Daten abgeleiteten physiologischen und sonstigen Daten werden für ein Individuum Vorschläge für Aktivitäten gemacht, welche die Erreichung des Ziels ermöglichen sollen.

Die Veröffentlichung WO 2012/008961 A1 beschreibt eine Methode und ein System, um auf einen bei einem Benutzer eines elektronischen Gerätes festgestellten Stress und/oder einen Mangel an Konzentration zu reagieren. Die Methode beinhaltet das Sammeln von Informationen bezüglich der Verwendung der elektronischen Vorrichtung durch den Benutzer, die Verarbeitung der Informationen und eine darauf basierende Bestimmung, ob der Benutzer Stress und/oder Mangel an Konzentration zeigt sowie bei Bejahung die Bereitstellung von Multimediainhalten, um dem entgegenzuwirken.

Die Veröffentlichung DE 10 2005 055 772 A1 beschreibt eine Personenwaage, die von einer zu wiegenden Person betretbar ist, wobei zumindest ein Handgriff vorgesehen ist, an dem sich die zu wiegende Person während des Betretens der Waage festhalten kann, wobei der Handgriff zumindest einen elektrischen und/oder optischen Messsensor aufweist, In einer Ausführungsform ist eine Auswerteeinheit zur Ermittlung eines Körpergewichtes und/oder eines Körperfettgehaltes und/oder einer Pulsfrequenz und/oder eines Blutdruckes der zu wiegenden Person vorgesehen.

Die Veröffentlichung JP 2011-072644 A beschreibt ein System und eine Methode zur Feststellung eines unnormalen Zustandes eines Gemütes oder Körpers, z.B. einer Depression.

Die Veröffentlichung US 6 484 062 B1 beschreibt ein Computersystem für die Stressbewältigung und ein Verfahren zu seinem Betrieb.

Vor diesem Hintergrund war es Aufgabe der vorliegenden Erfindung, ein Hausgerät mit einer Steuereinrichtung und einer berührungsempfindlichen Bedieneinrichtung bereitzustellen, das von einem Benutzer selbst für den Fall einer großen Auswahl an im Hausgerät durchführbaren Programmen und Einstellmöglichkeiten auf einfache Weise sowie rascher und präziser gesteuert werden kann. Im Ergebnis soll das Hausgerät insgesamt effizienter betrieben werden können. Erfindungsgemäß soll dabei der individuelle Zustand eines Benutzers des Hausgerätes berücksichtigt werden können.

Die Lösung dieser Aufgabe wird erfindungsgemäß erreicht durch ein Hausgerät gemäß Patentanspruch 1. Bevorzugte Ausführungsformen des erfindungsgemäßen Hausgeräts sind in den entsprechenden abhängigen Patentansprüchen aufgeführt. Bevorzugten Ausführungsformen des erfindungsgemäßen Hausgeräts entsprechen bevorzugte Ausführungsformen des erfindungsgemäßen Verfahrens und umgekehrt, auch wenn dies hierin nicht explizit festgestellt wird.

Gegenstand der Erfindung ist somit ein Hausgerät mit einer Steuereinrichtung und einer berührungsempfindlichen Bedieneinrichtung gemäß Patentanspruch 1, wobei das Hausgerät mindestens einen Sensor aufweist, mit dem mindestens eine Eigenschaft einer externen Eingabeeinheit, welche die Bedieneinrichtung berührt oder sich in einem Abstand x von dieser befindet, wobei x kleiner ist als ein vorgegebener maximaler Abstand xₘₐₓ, gemessen werden kann, wobei die Steuereinrichtung eingerichtet ist, um die mindestens eine gemessene Eigenschaft in Hinblick auf die Art und/oder den Zustand der externen Eingabeeinheit auszuwerten und das Hausgerät in Abhängigkeit von Art und/oder Zustand der externen Eingabeeinheit zu steuern.

Erfindungsgemäß ist die externe Eingabeeinheit ein menschlicher Körper, ein Teil eines menschlichen Körpers oder ein von einem menschlichen Körperteil geführter länglicher Eingabegegenstand.

Vorzugsweise ist die externe Eingabeeinheit ein länglicher Eingabegegenstand, dessen eines Ende hinsichtlich Material, Fläche und/oder Form so ausgestaltet ist, dass mit dem mindestens einen Sensor mindestens eine Eigenschaft in Hinblick auf die Art und/oder den Zustand der externen Eingabeeinheit ausgewertet werden kann, so dass das Hausgerät in Abhängigkeit von der ermittelten Art und/oder dem Zustand der externen Eingabeeinheit gesteuert werden kann. Besonders bevorzugt ist die externe Eingabeeinheit ein Stift. Die Verwendung eines länglichen Eingabegegenstandes ermöglicht es auch einer Person, die hinsichtlich ihrer körperlichen Ausdrucksmöglichkeiten eingeschränkt ist, ein Hausgerät unter Berücksichtigung ihres Zustandes, insbesondere ihres gesundheitlichen und/oder emotionalen Zustandes, zu steuern. Beispielsweise könnte für den Betrieb eines Hausgerätes ein Satz von verschiedenen länglichen Eingabegegenständen vorgesehen sein, die sich hinsichtlich ihrer Eigenschaften unterscheiden. Beispielsweise könnten sich die länglichen Eingabegegenstände in ihrem Widerstand oder in ihrer Berührungsfläche, d.h. der Größe der Fläche, über welche die externe Eingabeeinheit die berührungsempfindliche Bedieneinrichtung berührt bzw. berühren kann, unterscheiden. Jedem dieser Eingabegegenstände könnte ein unterschiedlicher gesundheitlicher Zustand oder ein Gemütszustand oder aber ein Stresszustand oder ein Zustand mangelnder Konzentrationsfähigkeit zugeordnet sein. Eine solche Ausführungsform setzt allerdings eine bewusste Entscheidung eines Benutzers des Hausgerätes voraus.

Der Sensor ist erfindungsgemäß nicht besonders eingeschränkt, solange er die erfindungsgemäß vorgesehene Aufgabe erfüllen kann. Insbesondere können sich die Messprinzipien unterscheiden. Außerdem kann es sich beim Sensor um einen einzelnen Sensor oder um ein Sensorsystem aus mehreren Sensoren handeln. Schließlich kann ein solches Sensorsystem auch aus einer Abstrahlungseinheit für eine Strahlung, insbesondere eine IR-Strahlung, sowie einer Empfangseinheit für die von der externen Eingabeeinheit reflektierte Strahlung, insbesondere reflektierte IR-Strahlung, handeln.

In einer bevorzugten Ausführungsform des Hausgerätes ist mindestens ein Sensor eine Kamera, die am Hausgerät angebracht ist, und die mindestens eine Eigenschaft eine Geste oder ein Gesichtsausdruck eines sich im Abstand x, wobei 0 < x < xₘₐₓ gilt, zur Kamera befindlichen menschlichen Körpers. Hierbei ist die Kamera vorzugsweise in einer im Wesentlichen gleichen Ebene wie die Bedieneinrichtung oder im Hausgerät hinter der Bedieneinrichtung angeordnet. Auch eine solche Ausführungsform setzt im Allgemeinen eine bewusste Entscheidung eines Benutzers des Hausgerätes voraus. Der Begriff "Geste" ist hier breit auszulegen. Es kann beispielsweise eine bestimmte Stellung von einer Hand oder von zwei Händen eines Benutzers sein oder aber eine Körperstellung, z.B. eine Strammstellung seitlich zur Kamera oder mit dem Rücken bzw. der Vorderseite des Benutzers zur Kamera hin. Der Gesichtsausdruck kann beispielsweise durch ein Lächeln, ein Lachen, einen traurigen Zustand oder eine Grimasse gekennzeichnet sein. Bei dieser Ausführungsform ist im Allgemeinen in der Steuereinrichtung eine Software hinterlegt, welche die vorgesehenen Gesten oder Gesichtsausdrücke erkennen und auswerten kann. Dabei kann vorzugsweise auch vorgesehen sein, dass der Benutzer einer externen Eingabeeinheit darüber informiert wird, dass zwar das Vorhandensein einer externen Eingabeeinheit erkannt wird, aber deren Benutzung durch den Benutzer keine Messung bzw. Auswertung zulässt und daher eine geänderte Benutzung zu erfolgen hat.

Erfindungsgemäß ist es im Übrigen bevorzugt, wenn die Eigenschaften einer nicht bewusst von einem Benutzer steuerbaren Eingabeeinheit vom Hausgerät gemessen und zur Steuerung ausgewertet werden können.

In einer weiteren bevorzugten Ausführungsform ist die externe Eingabeeinheit eine menschliche Hand oder ein Teil davon und die mindestens eine Eigenschaft umfasst eine Größe einer Berührungsfläche auf der berührungsempfindlichen Bedieneinrichtung und/oder eine oder mehrere biophysikalische Eigenschaften des menschlichen Körpers oder eines Teils davon. Dabei sind die eine oder mehreren biophysikalischen Eigenschaften vorzugsweise ausgewählt aus der Gruppe, die aus Körpertemperatur, Puls, Elektrokardiogramm, Blutdruck, Feuchtigkeit, Glucosegehalt im Blut, Sauerstoffgehalt im Körper oder im Blut, Fettanteil und elektrischem Widerstand besteht. Hierbei bezieht sich der Begriff "Feuchtigkeit" insbesondere auf Schweiß, vor allem Handschweiß. Dieser kann ein Maß für einen Gesundheits- oder Stresszustand eines Benutzers sein.

Die gemessenen biophysikalischen Eigenschaften können in einer Ausführungsform der Erfindung auch zu einer Identifizierung eines Benutzers herangezogen werden. Bei dieser Ausführungsform sind vorteilhaft für diesen Benutzer in der Steuereinrichtung des Hausgerätes bestimmte Angaben hinterlegt, beispielsweise Zahlenwerte für die biophysikalischen Eigenschaften, welche verschiedenen gesundheitlichen und/oder emotionalen Zuständen des Benutzers zugeordnet werden können. Es können auch Vorlieben oder Wünsche des Benutzers für die Betriebsweise des Hausgerätes hinterlegt sein.

Die Messung von biophysikalischen Eigenschaften ist dem Fachmann an sich bekannt. Zur Messung können je nach zumessender Eigenschaft Sensoren verwendet werden, die auf eine Berührung reagieren, d.h. berührungsempfindliche Sensoren, wobei gegebenenfalls nicht nur der Finger einer Hand zur Berührung ausreicht, sondern auch ein Finger der zweiten Hand zur Messung herangezogen werden muss. Dann erfolgt vorzugsweise durch eine Anzeigevorrichtung des Hausgerätes ein entsprechender Hinweis an den Benutzer. So kann zur Detektion des Pulsschlages eines Benutzers die vom Herzen des Benutzers erzeugte Spannung zwischen den beiden Händen detektiert werden. Dies kann beispielsweise dadurch geschehen, dass zwei Finger von zwei Händen auf hierzu vorgesehenen Kontaktflächen der berührungsempfindlichen Bedieneinrichtung platziert werden.

Es können aber auch Sensoren verwendet werden, die eine elektromagnetische Strahlung aussenden und eine reflektierte Strahlung messen. Beispielsweise könnte ein IR-Sensor verwendet werden, der einen IR-Strahler und eine IR-Empfangseinrichtung umfasst. Weist nämlich der Sensor eine Lichtquelle wie beispielsweise eine LED (Light Emitting Diode) oder einen Laser auf, insbesondere einen Halbleiterlaser, kann dieser beispielsweise zur Messung des Pulses herangezogen werden. Somit können beispielsweise der Pulsschlag des Benutzers und damit die Pulsfrequenz auch optisch bestimmt werden. Dies kann beispielsweise dadurch erfolgen, dass die sich durch den Pulsschlag ändernde Rückstreueigenschaft bzw. die sich mit der Zeit durch den Pulsschlag ändernde Reflektivität des menschlichen Gewebes gemessen und ausgewertet wird.

Überdies ist es bei einer Kombination verschiedener Sensoren beispielsweise möglich, über die zeitliche Verschiebung eines elektrisch gemessenen Spannungssignals des Pulsschlages und einem optisch gemessenen Signal des Pulsschlages den Blutdruck des Benutzers zu messen.

Schließlich kann ein IR-Sensor (Infrarotsensor) auch zur Messung der Körpertemperatur eines Benutzers verwendet werden. Bei einer solchen Ausführungsform ist beispielsweise in der berührungsempfindlichen Bedieneinrichtung ein Infrarotsensor vorhanden, der aus einer Infrarotquelle, d.h. einem IR-Strahler, und einem Infrarotempfangsgerät, d.h. einer IR-Empfangseinrichtung, besteht.

Im Übrigen kann bei Vorhandensein eines solchen optischen Sensors auch ein Photoplethysmogramm aufgenommen werden, um das Venensystem eines Menschen zu untersuchen und beispielsweise den Blutdruck zu messen. Die Photoplethysmographie, auch als Licht-Reflexions-Rheographie bezeichnet, ist eine nichtinvasive Screeningmethode zur Funktionsbeurteilung des Venensystems der Extremitäten eines Menschen, z.B. eines Fingers. Dabei wird Infrarotlicht einer definierten Wellenlänge mit einem Absorptionsmaximum im nahen infraroten Bereich in die Haut eingestrahlt und anschließend der reflektierte Anteil registriert und aufgezeichnet. Der Anteil des reflektierten Infrarot-Lichtes ist abhängig vom Füllzustand der kutanen Venenplexus, der sich während eines Bewegungsprogrammes ändert. Eine solche nichtinvasive Messung des Blutdrucks ist beispielsweise in der DE 10043266 A1 beschrieben.

In einer besonders bevorzugten Ausführungsform enthält das Hausgerät eine optische und/oder akustische Anzeigevorrichtung. Dabei ist diese Anzeigevorrichtung vorzugsweise eingerichtet, um einen Benutzer darüber zu informieren, inwieweit die mindestens eine Eigenschaft der externen Eingabeeinheit gemessen und ausgewertet werden kann. So könnte beispielsweise ein Hausgerät möglicherweise zwar die Anwesenheit einer externen Eingabeeinheit feststellen, aber möglicherweise nicht deren Eigenschaften messen, weil in Ausführungsformen der Erfindung beispielsweise das Ausmaß einer Berührung der berührungsempfindlichen Bedieneinrichtung nicht ausreichend ist oder eine Geste zu undeutlich ist. Es könnte dann auch vorgesehen sein, dass die Anzeigeeinheit eine Empfehlung an den Benutzer des Hausgerätes ausgibt, wie die externe Eingabeeinheit zu benutzen ist.

Die berührungsempfindliche Bedieneinrichtung im erfindungsgemäßen Hausgerät ist nicht eingeschränkt. So kann eine auf einer Widerstandsänderung, einer Druckänderung oder auf einer Kapazitätsänderung beruhende berührungsempfindliche Bedieneinrichtung verwendet werden. Erfindungsgemäß bevorzugt ist die Verwendung einer kapazitiven Bedieneinrichtung. Die berührungsempfindliche Bedieneinrichtung weist mindestens eine berührungsempfindliche Fläche auf, über welche eine Steuerung des Hausgeräts erfolgt. Die Größe dieser Fläche, die hierin auch als Kontaktfläche bezeichnet wird, ist im Allgemeinen so gewählt, dass die Berührung durch einen Finger, eine Hand oder einen Eingabegegenstand gut erfasst werden kann. Dabei können die erfindungsgemäß vorgesehenen Sensoren in solchen berührungsempfindlichen Flächen, d.h. Kontaktflächen, integriert sein oder aber als davon unabhängige Kontaktflächen vorliegen. Je nach zu messender biophysikalischer Eigenschaft können eine oder mehrere Kontaktflächen für die Messung einer biophysikalischen Eigenschaft vorgesehen sein.

In einer bevorzugten Ausführungsform des Hausgerätes umfassen Art und/oder Zustand der externen Eingabeeinheit einen Gesundheitszustand, einen Konzentrationsmangel, einen Stresszustand und/oder einen negativen oder positiven Gemütszustand. Ein negativer Gemütszustand wäre dabei beispielsweise ein depressive Stimmung oder ein Zustand erhöhter Reizbarkeit. Hierbei können solche Zustände auch in Gruppen zusammengefasst werden, so dass eine verbesserte übersichtliche Steuerung des Hausgerätes möglich ist. "Übersichtliche Steuerung" bedeutet beispielsweise, dass einer Zustandsgruppe eine bestimmte im Hausgerät durchzuführende Maßnahme in der Steuereinheit zugeordnet ist.

Vorzugsweise ist nämlich die Steuereinrichtung eingerichtet, um das Hausgerät so zu steuern, dass die Durchführung eines Arbeitsprogrammes im Hausgerät und/oder die Bedienbarkeit der berührungsempfindlichen Bedieneinrichtung hinsichtlich Menüauswahl und Gestaltung in Abhängigkeit von Art und/oder Zustand der externen Eingabeeinheit erfolgt. Das Arbeitsprogramm sowie die Ausgestaltung der berührungsempfindlichen Bedieneinrichtung werden dabei vom Hausgerät abhängen. Zur Ausgestaltung der berührungsempfindlichen Bedieneinrichtung gehören jedenfalls die Komplexität oder Einfachheit der Auswahl von im Hausgerät durchführbaren Programmen, sowie Größe, Farbe, Helligkeitswert und/oder Kontrastwert einer gewählten Darstellung.

Das Hausgerät ist erfindungsgemäß nicht besonders eingeschränkt. Besonders bevorzugt weist das Hausgerät allerdings zahlreiche Möglichkeiten für ein darin auszuführendes Programm auf, so dass die Benutzung für einen Benutzer des Hausgerätes kompliziert sein kann. Dann erweist sich die vorliegende Erfindung als besonders vorteilhaft.

Ein Beispiel für ein erfindungsgemäßes Hausgerät ist ein Hausgerät, in dem ein Lebensmittel zubereitet wird, beispielsweise ein Ofen, oder ein Hausgerät, in dem ein Lebensmittel vorgehalten wird, z.B. ein Kühlschrank.

Das erfindungsgemäße Hausgerät kann insbesondere auch ein wasserführendes Hausgerät sein, mit einem Behälter zur Aufnahme von zu behandelnden Gegenständen, das ausgewählt ist aus der Gruppe, die aus einer Waschmaschine, einem Waschtrockner, einem Trockner und einem Geschirrspülgerät besteht.

Das erfindungsgemäße Hausgerät verfügt im Allgemeinen abhängig von seiner Art über mindestens einen weiteren Sensor zur Bestimmung einer für den Betrieb des Hausgerätes wichtigen Eigenschaft. Ist das Hausgerät beispielsweise eine Waschmaschine, so könnten geeignete Sensoren Art und Menge der Beladung mit Wäsche und deren Verschmutzungsgrad ermitteln. Bei einem Waschtrockner könnten die Sensoren beispielsweise neben Art und Menge der Beladung mit Wäsche auch die Feuchte der Wäsche ermitteln. Anhand solcher Daten könnte die Steuereinrichtung bestimmte Verfahrensweisen bewerten und diese Bewertung dann in die Bedienung des Hausgerätes mittels der berührungsempfindlichen Bedieneinrichtung einfließen lassen. Beispielsweise könnte die Steuereinrichtung so eingerichtet sein, dass die Auswahl von Programmvorgaben, die von der Steuereinrichtung als für das Hausgerät nachteilig angesehen werden, erschwert wird oder nachteilige Programmvorgaben als solche für einen Benutzer gekennzeichnet werden.

In einer erfindungsgemäß bevorzugten Ausführungsform ist das Hausgerät eingerichtet, um bei Feststellung eines kranken Zustandes eines Benutzers ein Programm, d.h. Arbeitsprogramm, mit verbesserter Hygienewirkung mit Vorrang durchzuführen. Das Arbeitsprogramm wird hierbei von der Art des Hausgerätes abhängen. Ein Programm mit einer Hygienewirkung wird insbesondere in einer Waschmaschine, einem Waschtrockner oder einem Trockner durchgeführt. Eine Verbesserung einer Hygienewirkung kann dann insbesondere über die Einstellung einer höheren Temperatur für ggf. einen längeren Zeitraum erreicht werden. Es können gegebenenfalls auch andere Maßnahmen wie die Zugabe einer desinfizierenden Substanz oder die Bestrahlung mit desinfizierenden Strahlen, z.B. UV-Licht, vorgesehen sein.

Ist das Hausgerät ein Kühlschrank, könnte einem Benutzer bei Feststellung eines kranken Zustandes und/oder einer Fettleibigkeit als Ergebnis der erfindungsgemäß vorgesehenen Messung und Auswertung eine Essempfehlung gegeben werden, wobei vorzugsweise in einem Speicher des Kühlschranks hinterlegt ist, welche Speisen und Getränke darin aufbewahrt werden.

In einer bevorzugten Ausführungsform des Hausgeräts zeigt die Bedieneinrichtung bei einem negativen Gemütszustand, einem Stresszustand oder bei einem Konzentrationsmangel eine vereinfachte Menüauswahl an und/oder die Gestaltung der Menüauswahl erfolgt bunter, mit veränderten Größen von Symbolen oder Schriftzeichen, mit einem veränderten Helligkeitswert und/oder einem veränderten Kontrastverhältnis. So könnte beispielsweise bei einem negativen Gemütszustand die Anzeige der Menüauswahl unter Verwendung von warmen und fröhlichen Farben erfolgen. So soll "Blau" beruhigend wirken. Es könnte auch für eine ruhigere Betriebsweise des Hausgerätes gesorgt werden, beispielsweise über eine Herabsetzung der Drehzahl bei einer Waschmaschine oder einem Wäschetrockner. Schließlich könnte eine akustische Anzeigevorrichtung auch für eine angenehme Geräuschkulisse, beispielsweise mit entspannender oder mit an(auf)regender Musik, sorgen.

Ist über die Messung der Informationen der externen Eingabeeinheit auch die Identität des Benutzers ermittelt worden, könnte bei einer Hinterlegung von Lieblingsfarben und/oder Lieblingsmusik des ermittelten Benutzers die Menüauswahl und die Anzeigevorrichtung darauf abgestimmt betrieben werden.

In einer weiteren bevorzugten Ausführungsform des Hausgeräts und insbesondere auch eines unten beschriebenen erfindungsgemäßen Verfahrens wird anhand der Steuereinrichtung ermittelt, ob eine gemessene Eigenschaft innerhalb eines zulässigen Bereiches liegt. Bei Feststellung, dass diese gemessene Eigenschaft außerhalb eines zulässigen Bereiches liegt, könnte ein weiterer Betrieb des Hausgerätes nicht möglich sein und/oder die Steuereinrichtung eingerichtet sein, um einen Alarm auszulösen. Hierbei ist der Begriff "Alarm" breit auszulegen. Es kann sich hierbei um eine optische und/oder akustische Alarmmeldung einer akustischen und/oder optischen Anzeigevorrichtung handeln. Alternativ oder in Ergänzung dazu könnte das Hausgerät eine Warnmeldung beispielsweise über eine Telefonleitung oder das Internet absetzen.

In einer bevorzugten Ausführungsform der Erfindung ist daher die Steuereinrichtung eingerichtet, um zu ermitteln, ob eine gemessene Eigenschaft innerhalb eines zulässigen Bereiches liegt, und um bei Feststellung, dass diese gemessene Eigenschaft außerhalb eines zulässigen Bereiches liegt, einen weiteren Betrieb des Hausgerätes zu unterbinden und/oder einen Alarm auszulösen.

In einer weiteren bevorzugten Ausführungsform ist die Steuereinrichtung dazu eingerichtet, die Berührung in Hinblick auf die Erkennung eines Benutzers und von dessen gespeicherten Vorlieben auszuwerten. Dies kann beispielsweise dadurch geschehen, dass im berührungsempfindlichen Zentrum eine Erkennung eines Fingerabdrucks vorgesehen ist. Nach der Zuordnung des Fingerabdrucks zu einer Person kann dann die weitere Menügestaltung auf dem Touchscreen, also die Abfolge von Programmclustern, Unterclustern usw., unter Berücksichtigung von in der Steuereinrichtung hinterlegten Vorlieben erfolgen.

Das Hausgerät, im Allgemeinen eine darin eingesetzte Steuereinrichtung, kann sich in Ausführungsformen der Erfindung dann die bevorzugten Entscheidungen eines Benutzers merken und bei einer späteren Benutzung des Hausgerätes heranziehen, um Entscheidungssituationen zu optimieren. Dies kann dadurch geschehen, dass in der berührungsempfindlichen Bedieneinrichtung beispielsweise spezifische Menüangebote gezeigt werden oder bevorzugte Programme und Optionen hervorgehoben werden.

Ferner offenbart ist ein Verfahren zum Betrieb eines Hausgerätes mit einer Steuereinrichtung und einer berührungsempfindlichen Bedieneinrichtung offenbart, wobei das Hausgerät mindestens einen Sensor aufweist, mit dem mindestens eine Eigenschaft einer externen Eingabeeinheit, welche die Bedieneinrichtung berührt oder sich in einem Abstand x von dieser befindet, wobei x kleiner ist als ein vorgegebener maximaler Abstand xₘₐₓ, gemessen werden kann, und wobei die Steuereinrichtung eingerichtet ist, um die mindestens eine gemessene Eigenschaft in Hinblick auf die Art und/oder den Zustand der externen Eingabeeinheit auszuwerten und das Hausgerät in Abhängigkeit von Art und/oder Zustand der externen Eingabeeinheit zu steuern, wobei mit dem mindestens einen Sensor mindestens eine Eigenschaft einer externen Eingabeeinheit, welche die Bedieneinrichtung berührt oder sich in einem Abstand x von dieser befindet, wobei x kleiner ist als ein vorgegebener maximaler Abstand xₘₐₓ, gemessen wird und die Steuereinrichtung die mindestens eine gemessene Eigenschaft in Hinblick auf die Art und/oder den Zustand der externen Eingabeeinheit auswertet und das Hausgerät in Abhängigkeit von Art und/oder Zustand der externen Eingabeeinheit steuert.

Die Erfindung hat den Vorteil, dass auf einfache und übersichtliche Weise ein Hausgerät gesteuert werden kann. Durch die Berücksichtigung der körperlichen und/oder geistigen Situation eines Benutzers und in Ausführungsformen von dessen Identität kann eine angepasste Auswahl an Arbeitsprogrammen angezeigt werden und/oder ein bestimmtes Arbeitsprogramm automatisch durchgeführt werden. In Ausführungsformen der Erfindung, bei denen die Identität des Benutzers ermittelt wird, kann bei Vorliegen gespeicherter Eigenschaften und Vorlieben des Benutzers eine noch bessere Anpassung der Betriebsweise des Hausgerätes erfolgen.

Dadurch ist der Betrieb eines Hausgerätes mit einer verbesserten Sicherheit, aber auch mit einer verbesserten Handhabbarkeit (Bedienerfreundlichkeit) möglich.

Dies schafft insbesondere auch eine stärkere emotionale Bindung eines Benutzers zum Hausgerät. Generell kann die Akzeptanz neuer Hausgeräte oder Bedienkonzepte verbessert werden.

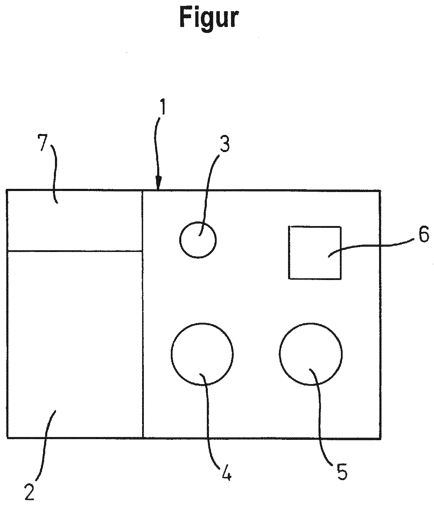

Die Erfindung wird im Folgenden anhand einer in der einzigen Figur gezeigten berührungsempfindlichen Bedieneinrichtung einer nicht einschränkenden Ausführungsform eines erfindungsgemäßen Hausgerätes illustriert.

Die Figur zeigt eine berührungsempfindliche Bedieneinrichtung 1 mit einem Anzeigefeld 2 mit einem Programmmenü. Im Anzeigefeld 2 können räumlich vor den Anzeigen von im Hausgerät durchführbaren Arbeitsprogrammen und Optionen dafür berührungsempfindliche Flächen, d.h. Kontaktflächen, angeordnet sein. Auf die Ausgestaltung des Anzeigefeldes wird hier ansonsten nicht näher eingegangen. Oberhalb des Anzeigefeldes 2 befindet sich außerdem eine akustische und/oder optische Anzeigevorrichtung 7. Neben der Möglichkeit der Ausgabe von akustischen Signalen kann hier im Unterschied zum Anzeigefeld 2 auch eine Information eines Benutzers über einen Betriebszustand des Hausgerätes erfolgen oder beispielsweise eine Aufforderung in Hinblick auf eine richtige Benutzung einer externen Eingabeeinheit. In der hier gezeigten Anzeigevorrichtung 7 befinden sich keine berührungsempfindlichen Kontaktflächen.

In der berührungsempfindlichen Bedieneinrichtung 1 der Figur sind vier Sensoren 3 bis 6 angeordnet, mit deren Hilfe mindestens eine Eigenschaft einer externen Eingabeeinheit, welche die Bedieneinrichtung 1 berührt oder sich in einem Abstand x von dieser befindet, wobei x kleiner ist als ein vorgegebener maximaler Abstand xₘₐₓ, gemessen werden kann. 3 ist hierbei eine Kamera zur Messung einer Geste oder eines Gesichtsausdruck eines Benutzers des Hausgerätes. 4 ist ein IR-Sensor, der hier nicht näher gezeigt einen IR-Strahler und eine IR-Empfangseinrichtung aufweist. Der IR-Sensor 4 kann wie oben beschrieben beispielsweise zur Messung einer Körpertemperatur oder eines Blutdruckes herangezogen werden. 4 bedeutet eine erste Kontaktfläche für eine externe Eingabeeinheit und beinhaltet einen ersten berührungsempfindlichen Sensor. 5 bedeutet eine zweite Kontaktfläche für eine externe Eingabeeinheit und beinhaltet einen zweiten berührungsempfindlichen Sensor. Sollen damit biophysikalische Eigenschaften gemessen werden, können den beiden Sensoren bzw. Kontaktflächen 5 und 6 unterschiedliche biophysikalische Eigenschaften zugeordnet sein. Einzelne biophysikalische Eigenschaften können allerdings auch erfordern, dass die beiden Sensoren bzw. Kontaktflächen 5 und 6 von möglichst entfernt voneinander liegenden Körperteilen berührt werden, z.B. von einem Finger der linken bzw. einem Finger der rechten Hand. Ggf. kann hierzu durch die Anzeigevorrichtung 7 eine Aufforderung an einen Benutzer ergehen.

### Bezugszeichenliste

- 1: Berührungsempfindliche Bedieneinrichtung, bspw. kapazitive Bedieneinrichtung
- 2: Anzeigefeld mit Programmmenü
- 3: Kamera
- 4: Erste Kontaktfläche für eine externe Eingabeeinheit; erster (berührungsempfindlicher) Sensor
- 5: Zweite Kontaktfläche für eine externe Eingabeeinheit; erster (berührungsempfindlicher) Sensor
- 6: IR-Sensor (umfassend einen IR-Strahler und eine IR-Empfangseinrichtung)
- 7: Akustische und/oder optische Anzeigevorrichtung

## Patentansprüche

1. Hausgerät mit einer Steuereinrichtung und einer berührungsempfindlichen Bedieneinrichtung (1), wobei das Hausgerät mindestens einen Sensor (2,3,4,5,6) aufweist, mit dem mindestens eine Eigenschaft einer externen Eingabeeinheit, welche die Bedieneinrichtung (1) berührt oder sich in einem Abstand x von dieser befindet, wobei x kleiner ist als ein vorgegebener maximaler Abstand xₘₐₓ, gemessen werden kann, wobei die Steuereinrichtung eingerichtet ist, um die mindestens eine gemessene Eigenschaft in Hinblick auf die Art und den Zustand der externen Eingabeeinheit auszuwerten und das Hausgerät in Abhängigkeit von Art und Zustand der externen Eingabeeinheit zu steuern, wobei der Zustand dem individuelle Zustand eines Benutzers des Hausgerätes entspricht, wobei die externe Eingabeeinheit ein menschlicher Körper, ein Teil eines menschlichen Körpers oder ein von einem menschlichen Körperteil geführter länglicher Eingabegegenstand ist.

2. Hausgerät nach Anspruch 1, **dadurch gekennzeichnet, dass** die externe Eingabeeinheit ein länglicher Eingabegegenstand ist, dessen eines Ende hinsichtlich Material, Fläche und/oder Form so ausgestaltet ist, dass mit dem mindestens einen Sensor (3,4,5,6) mindestens eine Eigenschaft in Hinblick auf die Art und/oder den Zustand der externen Eingabeeinheit ausgewertet werden kann, so dass das Hausgerät in Abhängigkeit von der ermittelten Art und/oder dem Zustand der externen Eingabeeinheit gesteuert werden kann.

3. Hausgerät nach Anspruch 2, **dadurch gekennzeichnet, dass** die externe Eingabeeinheit ein Stift ist.

4. Hausgerät nach Anspruch 1, **dadurch gekennzeichnet, dass** mindestens ein Sensor (3,4,5,6) eine Kamera (3) ist, die am Hausgerät angebracht ist, und die mindestens eine Eigenschaft eine Geste oder ein Gesichtsausdruck eines sich im Abstand x, wobei 0 < x < xₘₐₓ gilt, zur Kamera (3) befindlichen menschlichen Körpers ist.

5. Hausgerät nach Anspruch 1, **dadurch gekennzeichnet, dass** die externe Eingabeeinheit eine menschliche Hand oder ein Teil davon ist und dass die mindestens eine Eigenschaft eine Größe einer Berührungsfläche (4,5) auf der berührungsempfindlichen Bedieneinrichtung (1) und/oder eine oder mehrere biophysikalische Eigenschaften des menschlichen Körpers oder eines Teils davon umfasst.

6. Hausgerät nach Anspruch 5, **dadurch gekennzeichnet, dass** die eine oder mehreren biophysikalischen Eigenschaften ausgewählt sind aus der Gruppe, die aus Körpertemperatur, Puls, Elektrokardiogramm, Blutdruck, Feuchtigkeit, Glucosegehalt im Blut, Sauerstoffgehalt im Körper oder im Blut, Fettanteil und elektrischem Widerstand besteht.

7. Hausgerät nach einem der Ansprüche 1 bis 6, **dadurch gekennzeichnet, dass** es eine optische und/oder akustische Anzeigevorrichtung (7) enthält.

8. Hausgerät nach Anspruch 7, **dadurch gekennzeichnet, dass** die Anzeigevorrichtung (7) eingerichtet ist, um einen Benutzer darüber zu informieren, inwieweit die mindestens eine Eigenschaft der externen Eingabeeinheit gemessen und ausgewertet werden kann.

9. Hausgerät nach einem der Ansprüche 1 bis 8, **dadurch gekennzeichnet, dass** Art und/oder Zustand der externen Eingabeeinheit einen Gesundheitszustand, einen Konzentrationsmangel, einen Stresszustand und/oder einen negativen oder positiven Gemütszustand umfassen.

10. Hausgerät nach einem der Ansprüche 1 bis 9, **dadurch gekennzeichnet, dass** die Steuereinrichtung eingerichtet ist, um das Hausgerät so zu steuern, dass die Durchführung eines Arbeitsprogrammes im Hausgerät und/oder die Bedienbarkeit der berührungsempfindlichen Bedieneinrichtung (1) hinsichtlich Menüauswahl und Gestaltung in Abhängigkeit von Art und/oder Zustand der externen Eingabeeinheit erfolgt.

11. Hausgerät nach Anspruch 10, **dadurch gekennzeichnet, dass** es eingerichtet ist, um bei Feststellung eines kranken Zustandes eines Benutzers ein Programm mit verbesserter Hygienewirkung mit Vorrang durchzuführen.

12. Hausgerät nach Anspruch 10 oder 11, **dadurch gekennzeichnet, dass** die Bedieneinrichtung (1) bei einem negativen Gemütszustand, einem Stresszustand oder bei einem Konzentrationsmangel eine vereinfachte Menüauswahl anzeigt und/oder die Gestaltung der Menüauswahl erfolgt bunter, mit veränderten Größen von Symbolen oder Schriftzeichen, mit einem veränderten Helligkeitswert und/oder einem veränderten Kontrastverhältnis.

13. Hausgerät nach einem der Ansprüche 1 bis 12, **dadurch gekennzeichnet, dass** die Steuereinrichtung eingerichtet ist, um zu ermitteln, ob eine gemessene Eigenschaft innerhalb eines zulässigen Bereiches liegt, und um bei Feststellung, dass diese gemessene Eigenschaft außerhalb eines zulässigen Bereiches liegt, einen weiteren Betrieb des Hausgerätes zu unterbinden und/oder einen Alarm auszulösen.

## Claims

1. Household appliance with a control facility and a touch-sensitive operating facility (1), wherein the household appliance has at least one sensor (2,3,4,5,6), with which it is possible to measure at least one property of an external input unit, which touches the operating facility (1) or is located at a distance x therefrom, where x is less than a predefined maximum distance xₘₐₓ, wherein the control facility is configured to evaluate the at least one measured property with regard to the kind and the state of the external input unit and to control the household appliance as a function of the kind and state of the external input unit, wherein the state corresponds to the individual state of a user of the household appliance, wherein the external input unit is a human body, a part of a human body or an elongated input object guided by a human body part.

2. Household appliance according to claim 1, **characterised in that** the external input unit is an elongated input object, the one end of which is embodied in terms of material, surface and/or shape such that the at least one sensor (3,4,5,6) can be used to evaluate at least one property with regard to the kind and/or the state of the external input unit, meaning that the household appliance can be controlled as a function of the ascertained kind and/or the state of the external input unit.

3. Household appliance according to claim 2, **characterised in that** the external input unit is a pen.

4. Household appliance according to claim 1, **characterised in that** at least one sensor (3,4,5,6) is a camera (3), which is attached to the household appliance, and the at least one property is a gesture or a facial expression of a human body located at a distance x, where 0 < x < xₘₐₓ, from the camera (3).

5. Household appliance according to claim 1, **characterised in that** the external input unit is a human hand or a part thereof and that the at least one property comprises a variable of a touch surface (4,5) on the touch-sensitive operating facility (1) and/or one or more biophysical properties of the human body or a part thereof.

6. Household appliance according to claim 5, **characterised in that** the one or more biophysical properties are selected from the group consisting of body temperature, pulse, electrocardiogram, blood pressure, humidity, glucose content in the blood, oxygen content in the body or in the blood, fat content and electrical resistance.

7. Household appliance according to one of claims 1 to 6, **characterised in that** it contains an optical and/or acoustic display apparatus (7).

8. Household appliance according to claim 7, **characterised in that** the display apparatus (7) is configured to inform a user as to the extent to which the at least one property of the external input unit can be measured and evaluated.

9. Household appliance according to one of claims 1 to 8, **characterised in that** the kind and/or state of the external input unit comprise a health state, a lack of concentration, a stress state and/or a negative or positive emotional state.

10. Household appliance according to one of claims 1 to 9, **characterised in that** the control facility is configured to control the household appliance such that the performance of an operating program in the household appliance and/or the operability of the touch-sensitive operating facility (1) takes place with regard to menu selection and being designed as a function of the kind and/or state of the external input unit.

11. Household appliance according to claim 10, **characterised in that** it is configured to perform a program with improved hygiene effect with priority when a user is assessed to be in an unwell state.

12. Household appliance according to claim 10 or 11, **characterised in that** in the event of a negative emotional state, a stress state or in the event of a lack of concentration, the operating facility (1) indicates a simplified menu selection and/or the design of the menu selection takes place in a more colourful manner, with modified sizes of symbols or characters, with a modified brightness value and/or a modified contrast ratio.

13. Household appliance according to one of claims 1 to 12, **characterised in that** the control facility is configured to ascertain whether a measured property lies within a permissible range, and if it is assessed that this measured property lies outside of a permissible range, to prevent further operation of the household appliance and/or to trigger an alarm.

## Revendications

1. Appareil ménager avec un dispositif de commande et un dispositif d'actionnement (1) tactile, dans lequel l'appareil ménager présente au moins un capteur (2, 3, 4, 5, 6), lequel permet de mesurer au moins une caractéristique d'une unité de saisie externe, qui effleure le dispositif d'actionnement (1) ou se trouve à une distance x de celui-ci, dans lequel x est inférieur à une distance maximale prescrite xₘₐₓ et peut être mesuré, dans lequel le dispositif de commande est configuré afin d'évaluer l'au moins une caractéristique mesurée quant à la nature et à l'état de l'unité de saisie externe et de commander l'appareil ménager en fonction de la nature et de l'état de l'unité de saisie externe, dans lequel l'état correspond à l'état individuel d'un utilisateur de l'appareil ménager, dans lequel l'unité de saisie externe est un corps humain, une partie d'un corps humain ou un objet de saisie longitudinal porté par une partie de corps humain.

2. Appareil ménager selon la revendication 1, **caractérisé en ce que** l'unité de saisie externe est un objet de saisie longitudinal, dont l'une des extrémités est agencée de telle façon en termes de matériau, de surface et/ou de forme que l'au moins un capteur (3, 4, 5, 6) permet d'évaluer au moins une caractéristique quant à la nature et/ou à l'état de l'unité de saisie externe, de sorte que l'appareil ménager peut être commandé en fonction de la nature et/ou de l'état établi(e) de l'unité de saisie externe.

3. Appareil ménager selon la revendication 2, **caractérisé en ce que** l'unité de saisie externe est un stylo.

4. Appareil ménager selon la revendication 1, **caractérisé en ce qu'**au moins un capteur (3, 4, 5, 6) est une caméra (3) apposée sur l'appareil ménager, et l'au moins une caractéristique est un geste ou une expression faciale d'un corps humain se trouvant à une distance x, où 0 < x < xₘₐₓ, par rapport à la caméra (3).

5. Appareil ménager selon la revendication 1, **caractérisé en ce que** l'unité de saisie externe est une main humaine ou une partie de main humaine et **en ce que** l'au moins une caractéristique comprend une taille d'une surface de contact (4, 5) sur le dispositif d'actionnement (1) tactile et/ou une ou plusieurs caractéristique(s) biophysique(s) du corps humain ou d'une partie de celui-ci.

6. Appareil ménager selon la revendication 5, **caractérisé en ce que** la ou les caractéristique(s) biophysique(s) est/sont sélectionnée(s) parmi le groupe composé de la température corporelle, du pouls, de l'électrocardiogramme, de la tension artérielle, de l'humidité, du taux de glucose dans le sang, du taux d'oxygène dans le corps ou dans le sang, de la teneur en graisse et de la résistance électrique.

7. Appareil ménager selon l'une des revendications 1 à 6, **caractérisé en ce qu'**il contient un dispositif d'affichage (7) visuel et/ou sonore.

8. Appareil ménager selon la revendication 7, **caractérisé en ce que** le dispositif d'affichage (7) est configuré afin d'informer un utilisateur de la mesure dans laquelle l'au moins une caractéristique de l'unité de saisie externe peut être mesurée et évaluée.

9. Appareil ménager selon l'une des revendications 1 à 8, **caractérisé en ce que** la nature et/ou l'état de l'unité de saisie externe comprennent un état de santé, un manque de concentration, un état de stress et/ou une humeur négative ou positive.

10. Appareil ménager selon l'une des revendications 1 à 9, **caractérisé en ce que** le dispositif de commande est configuré afin de commander l'appareil ménager de sorte que la réalisation d'un programme de travail dans l'appareil ménager et/ou la manoeuvrabilité du dispositif d'actionnement (1) tactile s'opère en ce qui concerne le choix de menu et la conception en fonction de la nature et/ou de l'état de l'unité de saisie externe.

11. Appareil ménager selon la revendication 10, **caractérisé en ce qu'**il est configuré afin de réaliser en priorité un programme à effet amélioré sur l'hygiène en constatant un état malade d'un utilisateur.

12. Appareil ménager selon la revendication 10 ou 11, **caractérisé en ce que** le dispositif d'actionnement (1) affiche, en présence d'une humeur négative, d'un état de stress ou d'un manque de concentration, un choix de menu simplifié et/ou la conception du choix de menu s'opère de façon plus colorée, avec des tailles de symboles ou de caractères modifiées, avec une valeur de clarté modifiée et/ou un rapport de contraste modifié.

13. Appareil ménager selon l'une des revendications 1 à 12, **caractérisé en ce que** le dispositif de commande est configuré afin de déterminer si une caractéristique mesurée se situe dans une plage admissible et afin d'empêcher une exploitation supplémentaire de l'appareil ménager et/ou de déclencher une alarme en constatant que la caractéristique mesurée se situe en dehors d'une plage admissible.
